# EUROPEAN PATENT APPLICATION

(11) **EP 4 198 877 A1**
(43) Date of publication of application: **21.06.2023**
(21) Application number: 22161164.3
(22) Date of filing: 09.03.2022
(51) Int. Cl.: G06T 5/00, G06T 11/00, A61B 6/00, G06N 3/02

(54) **DENOISING PROJECTION DATA PRODUCED BY A COMPUTED TOMOGRAPHY SCANNER**

(30) Priority: 20.12.2021 US 202163291496 P
(71) Applicant: Koninklijke Philips N.V., 5656 AG Eindhoven (NL)
(72) Inventor: WUELKER, Christian, Eindhoven (NL); WILD, Sebastian, Eindhoven (NL); GRAß, Michael, Eindhoven (NL)
(74) Representative: Philips Intellectual Property & Standards

(57) **Abstract**

A mechanism for generating denoised basis projection data. Low-energy projection data and high-energy projection data is processed using a neural network that is trained to perform the dual task of decomposition and denoising. The neural network thereby directly outputs basis projection data, taking the place of existing decomposition and denoising techniques.

## Description

### TECHNICAL FIELD

The present disclosure relates to the field of medical imaging and, in particular, to the field of denoising and spectral material decomposition of projection data produced by a computed tomography scanner.

### BACKGROUND INFORMATION

Computed tomography (CT) scanners are well-established medical imaging devices that use a detector system or detector array to detect an interaction between X-ray radiated energy and irradiated material in order to generate medical imaging data.

In the field of computed tomography imaging, the use of non-traditional imaging techniques, such as spectral computed tomography, is becoming increasingly common. CT scanners that employ spectral computed tomography techniques are able to generate images that represents a particular type and/or frequency of particles incident at the detector system of the CT scanner, e.g. depending upon the modality of the CT scanner. These images are often called spectral images and/or material images, which can be derived from a set of so-called basis images.

One recent development in the field of CT scanners is a dual-layer CT scanner. A dual-layer CT scanner employs a detector system having a first layer of detectors to collect low-energy data and a second layer of detectors to collect high-energy data, the first and second layers being stacked. The low-energy data and the high-energy data is collected at a same time. Projection-space decomposition is used to process the low-energy data and the high-energy data to generate basis projection data. Usually, the basis projection data consist of photoelectric effect projection data and Compton-scatter projection data. The basis projection data can be reconstructed to form basis images, which can then be further processed to generate a variety of spectral images.

However, existing decomposition techniques operate on a pixel-by-pixel basis. This means that the decomposition of the low-energy and high-energy data into basis projection data is highly sensitive to noise. A conventional approach to reducing this noise is to perform pre-decomposition denoising (PDDN), and is often seen as an essential part of generating spectral images for using a dual-layer CT scanner. Due to the sensitivity of the decomposition approach to noise, often a large number of additional denoising steps are required after performing the decomposition, e.g. further denoising of the basis projection data and/or further denoising in the image domain.

There is an ongoing desire to improve the image quality produced by CT scanners.

### SUMMARY

The invention is defined by the claims.

According to examples in accordance with an aspect of the invention, there is provided a computer-implemented method of generating denoised basis projection data.

The computer-implemented method comprises: obtaining a low-energy projection dataset, comprising low-energy projection data generated for a plurality of different views of an imaged subject, wherein the low-energy projection data is generated by a first dual-layer CT scanner using a first layer of detectors; obtaining a high-energy projection dataset, comprising high-energy projection data generated for the same plurality of different views of the imaged subject, wherein the high-energy projection data is generated by the first dual-layer CT scanner using a second layer of detectors; and processing the low-energy projection dataset and the high-energy projection dataset using a neural network, trained to simultaneously perform denoising and material decomposition of the low-energy projection dataset and the high-energy projection dataset, to produce a plurality of sets of denoised basis projection data, each set providing a different type of spectral basis projection data at one of the plurality of different views of the imaged subject.

The proposed approach uses a neural network to perform both denoising and decomposition. In particular, the neural network is able to perform decomposition that is not performed on a pixel-by-pixel basis (of the detector response). This provides a more stable material decomposition process and improves a signal-to-noise ratio of the denoised basis projection data, i.e. reduces noise.

Embodiments provide a number of advantages, including an advantage that it would no longer be necessary to perform pre-processing denoising by PDDN, because the neural network decomposition approach would inherently be robust against noise (i.e. itself performs denoising). This significantly reduces an amount of processing that needs to be performed on the projection data, preserving features that may otherwise be mistaken as noise.

Moreover, because a neural network has a receptive field of view significantly larger than a single pixel, the proposed approach provides a decomposition technique that is stabilized by taking into account neighboring detector pixels. Use of different views of the imaged subject provides more robust denoising and, in particular, denoising of non-fixed pattern noise (i.e. noise not attributable to fixed sensor noise).

The proposed approach improves the denoising of basis projection data. This means that low-dose CT scanning techniques can be used reliably, as additional noise inherently present from low-dose CT scanning techniques can be mitigated using the improved denoising technique.

Thus, in some preferred examples, the low-energy projection dataset and the high-energy projection data set are generated by a CT scanner operating in a low-dose mode of operation. In other words, the low-energy projection dataset may be a low-dose, low-energy projection dataset, and the high-energy projection dataset may be a low-dose, high-energy projection dataset.

The plurality of sets of denoised basis projection data may comprise a set comprising photoelectric effect projection data and a set comprising Compton-scatter projection data.

The plurality of sets of denoised basis projection data may comprise only the set comprising photoelectric effect projection data and the set comprising Compton-scatter projection data.

The plurality of sets of denoised basis projection data may comprise: a first set comprising denoised basis projection data at a first subset of one or more views of the imaged subject; and a second set comprising denoised basis projection data at a second subset of one or more views of the imaged subject, the first subset of the views being different to the second subset of the views.

Optionally, the first set comprises photoelectric effect projection data and the second set comprises Compton-scatter projection data.

In preferred examples, the first subset of the views or the second subset of the views contains the centermost view of the plurality of different views. This approach ensures that the low-frequency content of other parts of the low- or high-energy projection data more closely corresponds to that of the output projection data. This improves an accuracy of denoising, as the processed projection data will more closely correspond to the target view of the projection data. The other of the first subset of the views or second subset of the views may contain a view immediately adjacent to the centermost view of the plurality of different views. This provides a similar improvement effect to the other of the first/second subset of the view.

As, in some examples, each subset of one or more views may contain only a single view, one of the first and second subset of the views may contain only the centermost view, and the other of the first and second subset of the views may contain only a view adjacent to the centermost view.

In some examples, the neural network is trained using a minimization approach that makes use of a regularization term. Use of a regularization term can reduce the amount of anticorrelation when producing the denoised basis projection data. The regularization term may be incorporated, for instance, in a loss function used to determine an error between a predicted output and a benchmark output provided by a training dataset.

In some examples, the neural network is trained using a first training dataset.

The first training dataset comprises: a first input training dataset formed of a plurality of first input training data entries that each comprise: a first example low-energy projection dataset comprising first low-energy projection data generated, by a first layer of detectors of a second dual-layer CT scanner operating in a low-dosage mode, for multiple views of a sample imaged subject; a first example high-energy projection dataset comprising first high-energy projection data generated, by a second layer of detectors of the second dual-layer CT scanner operating in a low-dosage mode, for the multiple views of the sample imaged subject.

The first training dataset also comprises a first output training dataset formed of a plurality of first output training data entries, each first output training data entry corresponding to a respective first input training data entry, and comprising: a plurality of first example sets of denoised basis projection data, wherein each first example set of denoised basis projection data is produced by processing, using a material decomposition process: a second example low-energy projection dataset comprising second low-energy projection data generated, by the first layer of detectors of the second dual-layer CT scanner operating in a high-dosage mode, for a first subset of the views of the sample imaged subject; and a second example high-energy projection dataset comprising second high-energy projection data generated, by the second layer of detectors of the second dual-layer CT scanner operating in a high-dosage mode, for a second subset of the views of the sample imaged subject.

In other examples, the neural network is trained using a second training dataset.

The second training dataset comprises: second output training dataset formed of a plurality of second output training data entries, each second output training data entry comprising a plurality of second example sets of projection data, wherein each second example set of projection data is produced by processing, using a material decomposition process: a third example low-energy projection dataset comprising third low-energy projection data generated, by a first layer of detectors of a second dual-layer CT scanner, for multiple views of a sample imaged subject; a third example high-energy projection dataset comprising third high-energy projection data generated, by a second layer of detectors of the second dual-layer CT scanner, for the multiple views of the sample imaged subject, wherein each second example set of projection data contains a subset of the multiple views of the sampled imaged subject.

The second training dataset also comprises a second input training dataset formed of a plurality of second input training data entries, each second input training data entry corresponding to a respective second output training data entry, and comprising: a fourth example low-energy projection dataset comprising fourth low-energy projection data generated by applying noise to the third low-energy projection data; and a fourth example high-energy projection dataset comprising fourth high-energy projection data generated by applying noise to the third high-energy projection data.

In some examples, the first subset of the views and/or the second subset of the views contains the centermost view of the multiple views. For instance, the first and/or second subset may contain only the centermost view of the multiple views.

The first single view may be different to the second single view. Of course, in this example, the first single view or the second single view is the centermost view of the multiple views. The other of the first single view or second single view may be immediately adjacent to the centermost view of the plurality of different views.

There is also proposed a computer program product comprising computer program code means which, when executed on a computing device having a processing system, cause the processing system to perform all of the steps of any herein described methods. The computer-program product is non-transitory.

There is also proposed a processing system configured to generate denoised basis projection data for a first dual-layer CT scanner that produces low-energy projection data using a first layer of detectors, and high-energy projection data using a second layer of detectors.

A device is configured to generate denoised basis projection data, the device comprising: processing circuitry; and a memory containing instructions that, when executed by the processing circuitry, configure the processing circuitry to: obtain a low-energy projection dataset, comprising low-energy projection data generated for a plurality of different views of an imaged subject, wherein the low-energy projection data is generated by a first dual-layer CT scanner using a first layer of detectors; obtain a high-energy projection dataset, comprising high-energy projection data generated for the same plurality of different views of the imaged subject, wherein the high-energy projection data is generated by the first dual-layer CT scanner using a second layer of detectors; and process the low-energy projection dataset and the high-energy projection dataset using a neural network, trained to perform denoising and material decomposition of the low-energy projection dataset and the high-energy projection dataset, to produce a plurality of sets of denoised basis projection data, each set providing a different type of spectral basis projection data at one of the plurality of different views of the imaged subject.

### BRIEF DESCRIPTION OF THE DRAWINGS

For a better understanding of the invention and to show more clearly how it may be carried into effect, reference will now be made, by way of example only, to the accompanying drawings, in which:
Figure 1 illustrates an imaging system in which embodiments may be implemented;
Figure 2 illustrates an approach adopted by embodiments;
Figure 3 is a flowchart illustrating a method according to an embodiment; and
Figure 4 illustrates a device according to an embodiment.

### DETAILED DESCRIPTION

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The disclosure provides a mechanism for generating denoised basis projection data. Low-energy projection data and high-energy projection data is processed using a neural network that is trained to perform the dual task of decomposition and denoising. The neural network thereby directly outputs basis projection data, taking the place of existing decomposition and denoising techniques.

Embodiments are based on the realization that a neural network is able to take account of information from different parts of projection data in performing a decomposition task, compared to existing approaches which operate on a pixel-by-pixel basis. This means that the neural network is simultaneously able to perform a denoising operation. Moreover, different views of projection data can be provided as input channels to the neural network, so that noise can be mitigated using information from other views to produce projection data at a target view.

Any herein described approach can be employed to process projection data produced by any suitable dual-layer CT scanner.

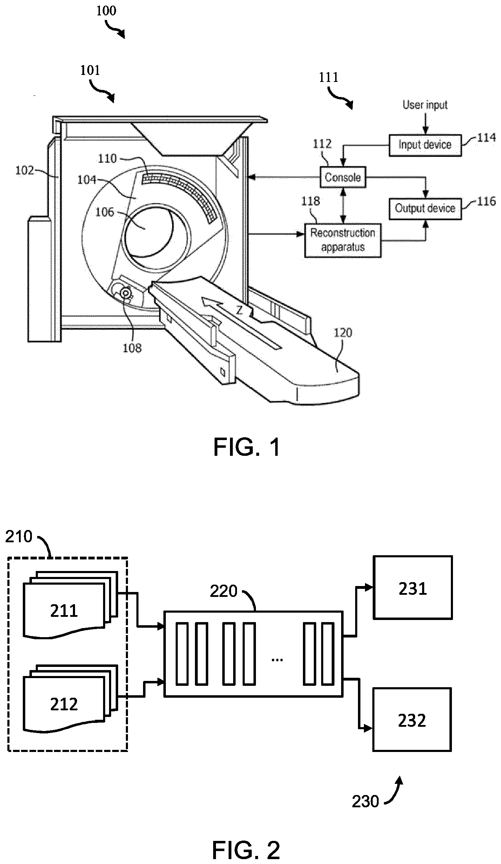

Figure 1 illustrates an imaging system 100, and in particular a computed tomography (CT) imaging system, in which embodiments of the present invention can be employed. The CT imaging system comprises a dual-layer CT scanner 101 and a processing interface 111, which processes and performs actions using data generated by the CT scanner 101.

The CT scanner 101 includes a generally stationary gantry 102 and a rotating gantry 104. The rotating gantry 104 is rotatably supported by the stationary gantry 102 and rotates around an examination region 106 about a longitudinal or z-axis.

A patient support 120, such as a couch, supports an object or subject such as a human patient in the examination region 106. The support 120 is configured to move the object or subject for loading, scanning, and/or unloading the object or subject.

A radiation source 108, such as an x-ray tube, is rotatably supported by the rotating gantry 104. The radiation source 108 rotates with the rotating gantry 104 and emits X-ray radiation that traverses the examination region 106.

A radiation sensitive detector array 110 subtends an angular arc opposite the radiation source 108 across the examination region 106. The detector array 110 includes two or more rows of detectors that extend along the z-axis direction, detects radiation traversing the examination region 106, and generates projection data indicative thereof. The projection data may, for instance, be cone-beam projection data. Projection data is data in the projection domain.

As the CT scanner is a dual-layer CT scanner, the detector array 110 comprises a first layer of detectors to collect low-energy projection data and a second layer of detectors to collect high-energy projection data, the first and second layers being stacked. The first layer of detectors is more proximate to the center of the CT scanner, i.e. to the radiation source, than the second layer of detectors. Low-energy projection data and the high-energy projection data is collected at a same time. A more complete description of an example of this technology for a dual-layer CT scanner is described in Rassouli, N., Etesami, M., Dhanantwari, A. et al. Detector-based spectral CT with a novel dual-layer technology: principles and applications. Insights Imaging 8, 589-598 (2017).

Other approaches and detector arrays for gathering low-energy projection data and high-energy projection data will be apparent to the skilled person and may include any form of detector array capable of generating projection data for different energy spectra (i.e. as used in any suitable spectral CT scanner).

As the rotating gantry rotates 104, the orientation or angle that the radiation source 108 makes with the stationary gantry 102 changes, thereby changing an angle that the radiation source 108 makes with the examination region 106 and an imaged subject positioned therein. This angle is known as an imaging angle. The detector array 110 will thereby generate low- and high-energy projection data for each of a plurality of different angles that the radiation source makes with the examination region. Each angle represents a different view of the imaged subject in the examination region. This produces low- and high- projection data for a plurality of different (projection) views of the imaged subject.

In the context of the present application, sets of projection (domain) data with different views indicates that the projection data represents different regions and/or imaging angles of the subject. Sets of projection (domain) data with the same view indicate that the sets of projection data represent a same region and imaging angle of the subject.

The processing interface 111 processes the projection data generated the CT scanner. The processing interface 111 may also facilitate user control over the operation of the CT scanner 102, e.g. controlling or defining an area or region to undergoing CT imaging and/or controlling a dosage or intensity of imaging.

In particular, the processing interface may comprise a general-purpose computing system or computer that serves as an operator console 112 and includes an input device(s) 114, such as a mouse, a keyboard, and/or the like and an output device(s) 116, such as a display monitor, a filmer or the like. The console 112 allows an operator to control operation of the system 100. Data generated by the processing interface can be displayed through at least one display monitor of the output device(s) 116.

The processing interface makes use of reconstruction apparatus 118 to process the projection data and reconstruct image data, e.g. for display at the output device 116 or for storage at an external server or database. It is to be appreciated that the reconstruction apparatus 118 can be implemented through a microprocessor(s), which executes a computer readable instruction(s) encoded or embedded on computer readable storage medium such as physical memory and other non-transitory medium. Additionally or alternatively, the microprocessor(s) can execute a computer readable instruction(s) carried by a carrier wave, a signal and other transitory or non-transitory medium.

In particular examples, the reconstruction apparatus 118 may first perform projection-space decomposition to produce basis projection data, which can be alternatively labelled basis projection domain data. For instance, projection-space decomposition can be performed to generate photoelectric effect projection data and/or Compton scatter projection data through appropriate processing of the low-energy projection data and high-energy projection data. Both photoelectric effect projection data and Compton-scatter projection data thereby provide examples of basis projection data.

More specifically, basis projection data is projection data that is responsive to a particular material type, in an imaged region of interest, or energy spectra/level of particles passing through the scanned/imaged region of interest during the CT scanning process. Different types of basis projection data are responsive to different types of material or energy spectra. Examples of different energy spectra include energy spectra associated with particles produced via a photoelectric effect or via Compton-scattering. Thus, examples of basis projection data include photoelectric effect projection data and Compton-scatter projection data. This is because particles generated as a result of different effects are of different energies, i.e. fall into different energy spectra. Examples of different materials include water, iodine, contrast agent and so on. These can be identified and distinguished from one another as different materials have different absorption characteristics. Thus, other examples of basis projection data include water projection data, iodine projection data, contrast agent projection data and so on.

Appropriate projection-space decomposition techniques, i.e. basis functions, for generating such basis projection data from low-energy projection data and high-energy projection data will be readily apparent to the skilled person. Typically, although not essentially, the basis projection data undergoes some filtering or denoising processes to produce denoised basis projection data.

The basis projection data or denoised basis projection data can then undergo reconstruction (e.g. spectral reconstruction) to generate basis image data. In existing approaches, the reconstruction apparatus 118 employs a filtered-backprojection (FBP) reconstruction, a reduced noise reconstruction algorithm such as an iterative reconstruction algorithm, which may operate in the image domain and/or projection domain, and/or other algorithm.

As with the basis projection data, different types of basis image data provide image data representative of different types of material and/or energy spectra in a scanned/imaged region of interest during the CT scanning process. By way of example, different types of basis image data include: photoelectric effect basis image data; Compton-scattering basis image data; iodine imaging data; water imaging data; contrast agent imaging data and so on.

The basis image data can be subsequently processed to produce a number of images relevant for diagnosis, such as Mono-energetic (MonoE) images, iodine maps, Z-effective images, and virtual non-contrast images. This could be performed by the reconstruction apparatus 118 or another processing system.

Of course, the reconstruction apparatus may also be used to generate single energy (SECT) or combined image data. Combined image data is generated from combined projection data, which is produced by combining the high and low-energy projection data together (e.g. summing or averaging the high and low-energy projection data).

The present disclosure relates to the generation of basis projection data from low-energy projection data and high-energy projection data, i.e. carrying out the decomposition process. In particular, proposed embodiments provide a technique for improved stability of the decomposition process, i.e. the generation of the basis projection data, and improved signal-to-noise of generated basis projection data. More specifically, the proposed techniques can replace the existing decomposition techniques, used to produce basis projection data, as well as any denoising techniques of basis projection data.

Embodiments thereby provide techniques for improving the reconstruction apparatus of a computed tomography system. By improving the signal-to-noise ratio of the basis projection data, lower dosages of radiation can be used to generate basis projection data of a same quality as previously available. This advantageously facilitates reduced exposure of the subject to radiation.

Figure 2 conceptually illustrates an exemplary approach according to an embodiment.

The approach proposes to process an input dataset 210, which is formed of a low-energy projection dataset 211 and a high-energy projection dataset 212, using a neural network 220 to produce a plurality 230 of sets or instances 231, 232 of denoised basis projection data 230. For example, the denoised basis projection data may comprise a set of photoelectric effect projection data 231 and/or a set of Compton scatter projection data 232.

The low-energy projection dataset 211 contains projection data generated from a first layer of detectors of the CT scanner, i.e. low-energy projection data. The high-energy projection data 212 contains projection data generated from a second layer of detectors of the CT scanner, i.e. high-energy projection data. The first and second layers of detectors are stacked, such that the first layer of detectors is more proximate to the center of the CT scanner, i.e. to the radiation source, than the second layer of detectors.

The low-energy projection data contains low-energy projection data obtained from a plurality of views, i.e. at different orientations of the radiation source with respect to the examination region. The high-energy projection data contains high-energy projection data obtained from the same plurality of views.

The neural network is appropriately trained to produce basis projection data, i.e. projection domain basis data. The basis projection data 230 includes at least two sets of denoised basis projection data, i.e. at least two instances of denoised basis projection data, in which different instances/sets correspond to different types of basis projection data. Thus, the neural network may produce, as output, at least two types of denoised basis projection data.

Suitable types include photoelectric effect projection data and Compton-scatter projection data. Other suitable types include water projection data, iodine projection data, contrast agent projection data and other material-based projection data. These latter types may provide projection data directed or tuned towards a specific material, as it is possible to identify or discriminate for a particular absorption characteristics, unique to a particular material, using projection data of different energy levels.

Whilst only two sets of denoised basis projection data are illustrated, it will be appreciated that more than two sets of denoised basis projection data may be output by the neural network.

Each set provides or contains a different type of spectral basis projection data at one of the plurality of different views by the low-energy and high-energy projection dataset. In other words, the denoised basis projection data of each set represents a particular target view of the subject.

The denoised basis projection data 230 contains a plurality of sets of denoised basis projection data. Each set may comprise denoised basis projection data at a single view, which is contrasted with the plurality of views contains in the projection data provided as input. In other examples, each set comprises denoised basis projection data for a subset, e.g. not all, of the views contained in the input dataset.

The neural network 220 simultaneously performs a decomposition process on the low- and high-energy projection data, as well as performing a denoising procedure. In particular, it is advantageously recognized that the decomposition performed by such a neural network will be inherently robust against noise, because decomposition is no longer performed on a pixel-by-pixel basis, rather all projection data is processed simultaneously. This means that information from other parts of the low- and high-energy projection data can be used to automatically compensate for noise.

Furthermore, because a neural network has a receptive field of view significantly larger than a single pixel, the proposed decomposition approach will be more stable, compared to approaches adopted by the prior art, by taking into account neighboring detector pixels.

The neural network 220 is preferably a convolutional neural network that makes use of one or more convolutional operations to process the low-energy projection data 211 and high-energy projection data 212. This may include ensuring that a convolution operation is performed in the row and column dimensions of the acquired data, as well as taking into account a given set of adjacent views in different feature channels. This ensures that the receptive field for each element of the denoised basis projection data is larger than a single picture and makes use of multiple dimensions of the input dataset, to provide more accurate and robust denoised basis projection data.

The approach makes use of a neural network to process the low- and high-energy projection data to produce the sets of denoised basis projection data.

The structure of a neural network is inspired by the human brain. Neural networks are comprised of layers, each layer comprising a plurality of neurons. Each neuron comprises a mathematical operation. In particular, each neuron may comprise a different weighted combination of a single type of transformation, e.g. the same type of transformation, sigmoid etc. but with different weightings. In the process of processing input data, the mathematical operation of each neuron is performed on the input data to produce a numerical output, and the outputs of each layer in the neural network are fed into the next layer sequentially. The final layer provides the output.

Methods of training neural networks are well known. Typically, such methods comprise obtaining a training dataset, comprising training input data entries and corresponding training output data entries. An initialized neural network is applied to each training input data entry to generate predicted output data entries. An error, which is produced by a loss or cost function, between the predicted output data entries and corresponding training output data entries is used to modify the machine-learning algorithm. Example loss functions include such as mean-squared error (MSE) or L1 error. This process can be repeated until the error converges, and the predicted output data entries are sufficiently similar, e.g. ±1%, to the training output data entries. This is commonly known as a supervised learning technique.

For example, weightings of the mathematical operation of each neuron may be modified until the error converges. Known methods of modifying a neural network include gradient descent, backpropagation algorithms and so on.

Thus, the neural network used for herein disclosed embodiments may be trained using a training dataset. Examples of particularly advantageous, but not essential, training datasets are hereafter described.

The training dataset may comprise an input training dataset formed of a plurality of input training data entries and an output training dataset formed of a plurality of output training data entries.

The output training dataset may include a plurality of output training data entries. Each output training data entry may comprise a plurality of sets of example basis projection data. Each set of example basis projection data is produced from: first low-energy projection data and first high-energy projection data. The first low-energy projection data is generated by a first layer of detectors of a second dual-layer CT scanner operating in a high-dosage mode. The first high-energy projection data is generated by the same second layer of detectors of the second dual-layer CT scanner operating in the high-dosage mode.

Producing the denoised basis projection data for the output training data entry may comprise processing this low-energy and high-energy projection data - obtained in a high-dosage mode of the CT scanner - using one or more standard decomposition techniques, and optionally performing noise filtering on the input and/or output of the decomposition technique(s).

The use of high-dosage projection data to generate the basis projection data for the output training data entries produces basis projection data with low noise, as high-dosage projection data will intrinsically have a relatively low noise due to relatively large amount of radiation used to detect/generate the projection data.

The input training dataset may include a plurality of input training data entries. Each input training entry corresponds to a different output training data entry, e.g. represents the same imaged region of the same subject as the corresponding output training data entry. Different sets of input and output training entries may correspond to different regions and/or different subjects. Indeed, this is preferred to avoid or reduce the risk of overfitting the neural network.

Each input training data entry comprises second low-energy projection data and second high-energy projection data.

In a first example, the second low-energy projection data is generated by the first layer of detectors of the second dual-layer CT scanner operating in a low-dosage mode. In this first example, the second high-energy projection data is generated by the second layer of detectors of the second dual-layer CT scanner operating in a low-dosage mode. The second low-energy and high-energy projection data corresponds spatially, i.e. to represent the same region/area of the same subject, as the first low-energy and high-energy projection data.

In a second example, the second low-energy projection data is generated by processing the first low-energy projection data used in generating the example basis projection data of the corresponding output training data entry. In this second example, the second high-energy projection data is generated by processing the first high-energy projection data used in generating the example basis projection data of the corresponding output training data entry. The processing may comprise adding noise, e.g. Gaussian noise, to the first low-energy/high-energy projection data. This can effectively synthesize or simulate low-energy or high-energy projection data accordingly, that would have been generated by the second CT scanner were it using a low-dosage mode.

The first example is beneficial for generating a neural network that more closely reflects real noise in low-dosage data. This provides a more accurate denoising neural network, particularly for use in denoising low-dosage data.

The second example is beneficial by exposing the sampled imaged subject(s) to less radiation when gathering the training dataset. This is because only a single imaging process, namely a single high dosage process, needs to be performed, rather than two imaging processes, namely a high dosage and a low dosage process, thereby exposing the subject(s) to less radiation.

It is further herein recognized that even when using high-dosage projection data in the generation of the training output data entries, there is likely to be anti-correlated noise present in the training output data entries.

One approach to reduce the amount of anti-correlated noise is to perform high-strength filtering of the high-dosage projection data in the training output data entries, thereby reducing the effect of noise in the training output data entries. However, this approach is not advisable, because this would result in undesirable training of the shortcomings of the current data processing chain into the neural network. Alternative approaches, that do not rely upon high-intensity filtering, would therefore be beneficial.

One approach is to modify the loss function, which is used when training the neural network, to include an additional regularization term. One option for this approach is to directly penalize anti-correlation in the neural network output for scatter and absorption. Another option would be to employ the same regularization term that is used in the anti-correlation filter (ACF) of known projection data processing algorithms, such as that proposed in European Patent no. EP 3,039,650.

A second technique makes use of the configuration of the neural network to process the plurality of views of the low-energy and high-energy projection data to produce the denoised basis projection data.

It has been previously explained how the neural network used in the disclosed examples is configured to receive, as input, low-energy and high-energy projection data generated for a plurality of views. The output of the neural network, on the other hand, may contain two or more sets of denoised basis projection data at only a single view for each set. Each set corresponds to a different type of basis projection data, e.g. photoelectric effect projection data or Compton-scatter projection data.

For the second technique, it is proposed to make the view or views associated with each set of denoised basis projection data different. In this way, each set contains denoised basis projection data for a different subset of the views, e.g. a different view. Thus, one of the sets of denoised basis projection data is associated with a first subset of the views, which may be a single first view. The other is associated with a second, different subset of the views, e.g. a single second view. When the training dataset is configured using this approach, it results in no or reduced anti-correlated noise in the output training data entries.

For improved consistency, this approach should be adopted in both the training and the inference phase of the neural network.

In preferred examples, in which each subset of views contains only a single view, the first view or the second view may be adjacent to one another. Thus, if the first view is a view i, the second view may be a view i+1 or a view i-1. For a typical CT scan, directly adjacent views are expected to have very similar low-frequency content, which makes the combined prediction of the adjacent views favorable, i.e. more accurate or robust, from the point of view of network training, as opposed to predicting two views which have a larger shift.

In some examples, the first view or the second view may be the centermost view of the available views in the projection data received as input by the neural network. Thus, if the plurality of views contained in the low- or high-energy projection data spans from j-n to j+n, then the first view or the second view may be j. This advantageously ensures that the content of the projection data in the low- or high-energy projection data are more likely to be similar, at least from a low-frequency content perspective, to the content in the denoised basis projection data output by the neural network.

Previously described examples make use of neural networks that process low-energy projection data and high-energy projection data. These two types of projection data are generated using two stacked detection layers of a dual-layer CT scanner.

In an extension to this approach, the dual-layer CT scanner may be configured to operate in a kVp switching mode, in which the radiation source of the CT scanner emits two distinct/different frequencies or frequency spectra of X-ray radiation. This emission may be performed alternately or sequentially.

In these scenarios, the low-energy projection data may comprise first low-energy projection data, which is generated when the first frequency or frequency spectra of X-ray radiation is output by the radiation source, and second low-energy projection data, which is generated when the second frequency or frequency spectra of X-ray radiation is output by the radiation source.

Similarly, the high-energy projection data may comprise first high-energy projection data, which is generated when the first frequency or frequency spectra of X-ray radiation is output by the radiation source, and second high-energy projection data, which is generated when the second frequency or frequency spectra of X-ray radiation is output by the radiation source.

Thus, the neural network would receive, for each view, four sections of projection data, instead of simply two. The neural network can still be appropriately trained to generate the sets of denoised basis projection data in the same manner.

The data corresponding to the two different kVp settings will contain complementary information about the absorption lengths at different energies. This improves the denoising capability of the neural network, as it will be able to make use of this information when combining information contained in these input channels for predicting the denoised basis projection data.

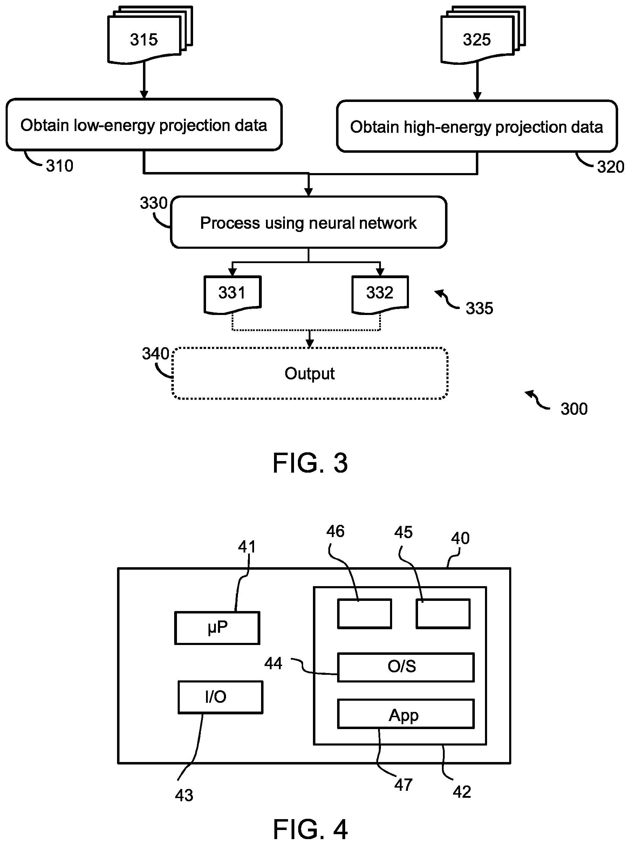

Figure 3 illustrates a method 300 according to an embodiment, which makes use of the above-described approaches. The method is computer-implemented, and may be performed by a processing system communicatively coupled to a CT scanner and/or a memory/storage unit.

The method 300 is configured to denoise basis projection data for a first dual-layer CT scanner that produces low-energy projection data using a first layer of detectors, and high-energy projection data using a second layer of detectors.

The method 300 comprises a step 310 of obtaining a low-energy projection dataset 315, comprising low-energy projection data generated for a plurality of different views of an imaged subject. This dataset may be obtained, for instance, from the CT scanner or the memory/storage unit.

The method 300 further comprises a step 320 of obtaining a high-energy projection dataset 325, comprising high-energy projection data generated for the same plurality of different views of the imaged subject. This dataset may similarly be obtained, for instance, from the CT scanner or the memory/storage unit.

The method 300 further comprises a step 330 of processing the low-energy projection dataset and the high-energy projection dataset using a neural network. The neural network is trained to perform denoising and material decomposition of the low-energy projection dataset and the high-energy projection dataset. Step 330 thereby produces a plurality 335 of sets of denoised basis projection data 331, 332, each set providing a different type of spectral basis projection data at one of the plurality of different views of the imaged subject. For example, the denoised basis projection data 330 may comprise a first set 331 of denoised basis projection data, e.g. photoelectric effect projection data 331, and a second set 332 of denoised basis projection data, e.g. Compton scatter projection data 332.

The method 300 may further comprise a step 340 of outputting the sets of denoised basis projection data, e.g. for further processing or reconstruction by a reconstruction apparatus. In particular, each output set of denoised basis projection data may be reconstructed into one or more basis images.

By way of further example, Figure 4 illustrates an example of a device 40, or a processing system, within which one or more parts of an embodiment may be employed. Various operations discussed above may utilize the capabilities of the device 40. For example, one or more parts of a system for generating sets of denoised basis projection data may be incorporated in any element, module, application, and/or component discussed herein. In this regard, it is to be understood that system functional blocks can run on a single computer or may be distributed over several computers and locations, e.g. connected via the internet.

The device 40 includes, but is not limited to, PCs, workstations, laptops, PDAs, palm devices, servers, storages, cloud computing devices, distributed processing systems and the like. Generally, in terms of hardware architecture, the device 40 may include one or more processing circuitries 41, memory 42, and one or more I/O devices 43 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processing circuitry 41 is a hardware device for executing software that can be stored in the memory 42. The processing circuitry 41 can be virtually any custom made or commercially available processing circuitry, a central processing unit (CPU), a digital signal processing circuitry (DSP), or an auxiliary processing circuitry among several processing systems associated with the device 40, and the processing circuitry 41 may be a semiconductor based microprocessing circuitry (in the form of a microchip) or a microprocessing circuitry.

The memory 42 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 42 may incorporate electronic, magnetic, optical, and/or other types of storage media. The memory 42 can have a distributed architecture, where various components are situated remote from one another, but can be accessed by the processing circuitry 41.

The software in the memory 42 may include one or more separate programs, each of which comprises an ordered listing of executable instructions for implementing logical functions. The software in the memory 42 includes a suitable operating system (O/S) 44, compiler 45, source code 46, and one or more applications 47 in accordance with exemplary embodiments. As illustrated, the application 47 comprises numerous functional components for implementing the features and operations of the exemplary embodiments. The application 47 of the device 40 may represent various applications, computational units, logic, functional units, processes, operations, virtual entities, and/or modules in accordance with exemplary embodiments, but the application 47 is not meant to be a limitation.

The operating system 44 controls the execution of other computer programs and provides scheduling, input-output control, file and data management, memory management, and communication control and related services. It is contemplated by the inventors that the application 47 for implementing exemplary embodiments may be applicable on all commercially available operating systems.

Application 47 may be a source program, executable program (object code), script, or any other entity comprising a set of instructions to be performed. When a source program, then the program is usually translated via a compiler, such as the compiler 45, assembler, interpreter, or the like, which may or may not be included within the memory 42, so as to operate properly in connection with the O/S 44. Furthermore, the application 47 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 43 may include input devices such as, for example but not limited to, a mouse, keyboard, scanner, microphone, camera, etc. Furthermore, the I/O devices 43 may also include output devices, for example but not limited to a printer, display, etc. Finally, the I/O devices 43 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 43 also include components for communicating over various networks, such as the Internet or intranet.

If the device 40 is a PC, workstation, intelligent device or the like, the software in the memory 42 may further include a basic input output system (BIOS). The BIOS is a set of essential software routines that initialize and test hardware at startup, start the O/S 44, and support the transfer of data among the hardware devices. The BIOS is stored in some type of read-only-memory, such as ROM, PROM, EPROM, EEPROM or the like, so that the BIOS can be executed when the device 40 is activated.

When the device 40 is in operation, the processing circuitry 41 is configured to execute software stored within the memory 42, to communicate data to and from the memory 42, and to generally control operations of the device 40 pursuant to the software. The application 47 and the O/S 44 are read, in whole or in part, by the processing circuitry 41, perhaps buffered within the processing circuitry 41, and then executed.

When the application 47 is implemented in software it should be noted that the application 47 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 47 can be embodied in any computer-readable medium for use by or in connection with an instruction execution system, apparatus, or device, such as a computer-based system, processing circuitry-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

In the context of the present disclosure, the I/O devices 43 may be configured to receive the input datasets from a medical imaging device. In another example, the input datasets are obtained from a memory arrangement or unit.

The I/O devices 43 may be configured to provide the output dataset(s) to a user interface. The user interface may be configured to provide a visual representation of the output dataset(s), e.g. display an image or images corresponding to the medical imaging data contained in the output dataset(s).

The skilled person would be readily capable of developing a device having processing circuitry for carrying out any herein described method. Thus, each step of the flow chart may represent a different action performed by the processing circuitry of a device, and may be performed by a respective module of the processing circuitry of the device.

Embodiments may therefore make use of a device. The device can be implemented in numerous ways, with software and/or hardware, to perform the various functions required. A processor is one example of a device which employs one or more microprocessors that may be programmed using software (e.g., microcode) to perform the required functions. A device may however be implemented with or without employing a processor, and also may be implemented as a combination of dedicated hardware to perform some functions and a processor, e.g., one or more programmed microprocessors and associated circuitry, to perform other functions.

Examples of device components that may be employed in various embodiments of the present disclosure include, but are not limited to, conventional microprocessors, application specific integrated circuits (ASICs), and field-programmable gate arrays (FPGAs).

In various implementations, a processor or device may be associated with one or more storage media such as volatile and non-volatile computer memory such as RAM, PROM, EPROM, and EEPROM. The storage media may be encoded with one or more programs that, when executed on processing circuitry, perform the required functions. Various storage media may be fixed within a processor or device or may be transportable, such that the one or more programs stored thereon can be loaded into a processor or device.

It will be understood that disclosed methods are preferably computer-implemented methods. As such, there is also proposed the concept of a computer program comprising code means for implementing any described method when said program is run on a device, containing processing circuitry, such as a computer. Thus, different portions, lines or blocks of code of a computer program according to an embodiment may be executed by a device or computer to perform any herein described method. In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented method of generating denoised basis projection data, the method comprising:
obtaining a low-energy projection dataset comprising low-energy projection data generated for a plurality of different views of an imaged subject, wherein the low-energy projection data is generated by a first dual-layer computed tomography (CT) scanner using a first layer of detectors;
obtaining a high-energy projection dataset comprising high-energy projection data generated for the same plurality of different views of the imaged subject, wherein the high-energy projection data is generated by the first dual-layer CT scanner using a second layer of detectors; and
processing the low-energy projection dataset and the high-energy projection dataset using a neural network trained to perform denoising and material decomposition of the low-energy projection dataset and the high-energy projection dataset, to produce a plurality of sets of denoised basis projection data, each set providing a different type of spectral basis projection data at a subset of the plurality of different views of the imaged subject.

2. The computer-implemented method of claim 1, wherein the plurality of sets of denoised basis projection data comprises a set comprising photoelectric effect projection data and a set comprising Compton-scatter projection data.

3. The computer-implemented method of claim 2, wherein the plurality of sets of denoised basis projection data comprises only the set comprising photoelectric effect projection data and the set comprising Compton-scatter projection data.

4. The computer-implemented method of any of claims 1 to 3, wherein the plurality of sets of denoised basis projection data comprises:
a first set comprising denoised basis projection data at a first subset of the views of the imaged subject; and
a second set comprising denoised basis projection data at a second subset of the views of the imaged subject, the first subset of the views being different to the second subset of the views.

5. The computer-implemented method of claim 4, wherein the first set comprises photoelectric effect projection data and the second set comprises Compton-scatter projection data.

6. The computer-implemented method of any of claims 4 or 5, wherein the first subset of the views or the second subset of the views includes the centermost view of the plurality of different views.

7. The computer-implemented method of claim 6, wherein the other of the first subset of the views or second subset of the views includes a view immediately adjacent to the centermost view of the plurality of different views.

8. The computer-implemented method of any of claims 1 to 7, wherein the neural network is trained using a minimization approach that makes use of a regularization term.

9. The computer-implemented method of any of claims 1 to 8, wherein the neural network is trained using a first training dataset, the first training dataset comprising:
a first input training dataset formed of a plurality of first input training data entries that each comprise:
a first example low-energy projection dataset comprising first low-energy projection data generated, by a first layer of detectors of a second dual-layer CT scanner operating in a low-dosage mode, for multiple views of a sample imaged subject;
a first example high-energy projection dataset comprising first high-energy projection data generated, by a second layer of detectors of the second dual-layer CT scanner operating in a low-dosage mode, for the multiple views of the sample imaged subject, and
a first output training dataset formed of a plurality of first output training data entries, each first output training data entry corresponding to a respective first input training data entry, and comprising:
a plurality of first example sets of denoised basis projection data, wherein each first example set of denoised basis projection data is produced by processing, using a material decomposition process:
a second example low-energy projection dataset comprising second low-energy projection data generated, by the first layer of detectors of the second dual-layer CT scanner operating in a high-dosage mode, for a first subset of the views of the sample imaged subject; and
a second example high-energy projection dataset comprising second high-energy projection data generated, by the second layer of detectors of the second dual-layer CT scanner operating in a high-dosage mode, for a second subset of the views of the sample imaged subject.

10. The computer-implemented method of any of claims 1 to 8, wherein the neural network is trained using a second training dataset, the second training dataset comprising:
a second output training dataset formed of a plurality of second output training data entries, each second output training data entry comprising a plurality of second example sets of projection data, wherein each second example set of projection data is produced by processing, using a material decomposition process:
a third example low-energy projection dataset comprising third low-energy projection data generated, by a first layer of detectors of a second dual-layer CT scanner, for multiple views of a sample imaged subject;
a third example high-energy projection dataset comprising third high-energy projection data generated, by a second layer of detectors of the second dual-layer CT scanner, for the multiple views of the sample imaged subject, wherein each second example set of projection data contains a subset of the multiple views of the sampled imaged subject, and
a second input training dataset formed of a plurality of second input training data entries, each second input training data entry corresponding to a respective second output training data entry, and comprising:
a fourth example low-energy projection dataset comprising fourth low-energy projection data generated by applying noise to the third low-energy projection data; and
a fourth example high-energy projection dataset comprising fourth high-energy projection data generated by applying noise to the third high-energy projection data.

11. The computer-implemented method of any of claims 1 to 10, wherein:
the first dual-layer CT scanner is configured to operate in a kVp switching mode, in which a radiation source of the first dual-layer CT scanner emits a first frequency or frequency spectra of X-ray radiation and a second frequency or frequency spectra of X-ray radiation,
the first frequency or frequency spectra of X-ray radiation is distinguishable from the second frequency or frequency spectra of X-ray radiation and wherein:
the low-energy projection data comprises first low-energy projection data, which is generated when a first frequency or frequency spectra of X-ray radiation is output by the radiation source, and second low-energy projection data, which is generated when a second frequency or frequency spectra of X-ray radiation is output by the radiation source; and/or
the high-energy projection data comprises first high-energy projection data, which is generated when the first frequency or frequency spectra of X-ray radiation is output by the radiation source, and second high-energy projection data, which is generated when the second frequency or frequency spectra of X-ray radiation is output by the radiation source.

12. A computer program product comprising computer program code means which, when executed on a computing device having a processing system, cause the processing system to perform all of the steps of the method according to any of claims 1 to 11.

13. A device configured to generate denoised basis projection data, the device comprising:
processing circuitry; and
a memory containing instructions that, when executed by the processing circuitry, configure the processing circuitry to:
obtain a low-energy projection dataset, comprising low-energy projection data generated for a plurality of different views of an imaged subject, wherein the low-energy projection data is generated by a first dual-layer CT scanner using a first layer of detectors;
obtain a high-energy projection dataset, comprising high-energy projection data generated for the same plurality of different views of the imaged subject, wherein the high-energy projection data is generated by the first dual-layer CT scanner using a second layer of detectors; and
process the low-energy projection dataset and the high-energy projection dataset using a neural network, trained to perform denoising and material decomposition of the low-energy projection dataset and the high-energy projection dataset, to produce a plurality of sets of denoised basis projection data, each set providing a different type of spectral basis projection data at one of the plurality of different views of the imaged subject.
